# EUROPÄISCHE PATENTSCHRIFT

(11) **EP 1 070 698 B1**
(45) Veröffentlichungstag und Bekanntmachung des Hinweises auf die Patenterteilung: **20.06.2007**
(21) Anmeldenummer: 00110808.3
(22) Anmeldetag: 22.05.2000
(51) Int. Cl.: C07C 29/145

(54) **Verfahren zur Hydrierung von Aceton**
Process for the hydrogenation of acetone
Procédé d'hydrogénation de l'acétone

(30) Priorität: 17.07.1999 DE 19933691
(43) Veröffentlichungstag der Anmeldung: 24.01.2001
(73) Patentinhaber: INEOS Phenol GmbH & Co. KG, 45966 Gladbeck (DE)
(72) Erfinder: Maschmeyer, Dietrich, Dr., 45657 Recklinghausen (DE); Schuler, Joachim, Dr., 45772 Marl (DE); Pompetzki, Werner, Dr., 46284 Dorsten (DE)
(74) Vertreter: polypatent

(56) Entgegenhaltungen:
- EP-A- 0 420 035
- WO-A-01/62692
- WO-A-99/03801
- SU-A- 1 460 920
- US-A- 5 495 055
- GRACHEV, A.M.; DATSEVICH, L.B.; KAKK, O.A.; NAGRODSKII, M.I.; OVCHINNIKOV, P.N.; RYLEEV, G.I.; GILAK, I.A.: "Development of a reactor for liquid-phase hydrogenation of acetone with liquid-phase recycle" DEVELOPMENT AND INVESTIGATION IN PROCESSES OF ORGANIC SYNTHESES, 1984, Seiten 1-3, XP002259856 -& GRACHEV, A.M.; ET AL.: DEVELOPMENT AND INVESTIGATION IN PROCESSES OF ORGANIC CHEMISTRY, Seiten 6-8, XP002261377

## Beschreibung

Die Erfindung betrifft ein Verfahren zur Hydrierung von Aceton zu Isopropanol.

Aceton ist ein großtechnisches Produkt und kann gezielt, z. B. durch Oxierung von Propen oder als Koppelprodukt bei der Hookschen Phenolsynthese gewonnen werden.

Bei der Hookschen Phenolsynthese werden pro Molekül Phenol ein Molekül Aceton erhalten. Die Absatzmöglichkeiten für Phenol und Aceton sind sehr unterschiedlich; so wird z. B. in der Synthese von Bisphenol-A Phenol und Aceton im Verhältnis 2 zu 1 verbraucht.

Ein mögliches Folgeprodukt des Acetons ist Isopropanol, das ein deutlich breiteres Anwendungsspektum aufweist. Ein recht bedeutender Anteil des Isopropanols wird zu Ethern, insbesondere Diisopropylether und tert.-Butylisopropylether verarbeitet.

Die Umwandlung von Aceton in Isopropanol erfolgt in der Regel durch katalytische Hydrierung. Für die Produktion von Isopropanolethern kommen meist Kombinationsverfahren aus Hydrierung und Veretherung zum Einsatz. So offenbaren EP 0 694 518, EP 0 665 207, EP 0 652 200 und EP 0 661 257 Verfahren zur Herstellung verschiedener Isopropylether. Gemeinsam ist in diesen Patentanmeldungen folgender Verfahrensablauf:
a) Katalytische Hydrierung einer acetonhaltigen, flüssigen Phase
b) Veretherung des so erhaltenen Isopropanols an sauren Katalysatorsystemen

Die Verfahrensschritte a) und b) werden unmittelbar hintereinander, d. h. ohne Aufarbeitung des aus a) erhaltenen Produktgemisches ausgeführt.

EP 0 665 207 lehrt darüber hinaus einen 1-Stufen-Prozeß, in dem a) und b) durch einen geeigneten Kombinationskatalysator in einem einzigen Reaktor durchgeführt werden.

Eine Isolierung des Isopropanols nach der Reaktionsstufe a) ist aufgrund der Nebenproduktbildung (die Verfahren sind auf die Herstellung der Isopropylether ausgelegt) sehr aufwendig.

Zur Herstellung von Isopropanol aus Aceton ist das in EP 0 379 323 beschriebene Verfahren geeigneter. Hier wird Aceton bei einer Temperatur von 20 bis 200 °C und Drücken von 1 bis 80 bar katalytisch hydriert, wobei zwingend ein Rieselreaktor eingesetzt wird. Rieselreaktoren werden eingesetzt, um eine hohe Stoffaustauschfläche zwischen Flüssigkeit und Gas zu schaffen. Sie müssen daher eine Rieselfläche mit großer Oberfläche aufweisen. Die Qualität des erhaltenen Isopropanols bzw. der Anteil an Nebenprodukten wird nicht diskutiert.

Für viele Anwendungszwecke darf Isopropanol keine Nebenprodukte wie Isopropylether oder Lösungsmittelspuren aus der Hydrierung enthalten. Insbesondere medizinische oder kosmetische Anwendungen oder Folgeprodukte des Isopropanols verlangen einen sehr hohen Reinheitsgrad. Hohe Reinheitsgrade sind großtechnisch nur mit aufwendigen Reinigungsschritten erreichbar. So können z. B. bei der Herstellung von Isopropanol durch Wasseranlagerung an Propen schwefelhaltige Verbindungen den Einsatz in der kosmetischen oder pharmazeutischen Industrie verhindern. Eine Entfernung dieser Komponenten ist nur durch eine Nachbehandlung des Isopropanols mit Aktivkohle, Al₂O₃ oder Metallen wie Kupfer oder Nickel möglich.

Es ist daher Aufgabe der vorliegenden Erfindung, ein Verfahren zur effizienten Hydrierung von Aceton zu hochreinem Isopropanol bereitzustellen. Überraschenderweise wurde gefunden, daß Aceton in einem Mehrstufenprozeß zu Isopropanol hoher Reinheit hydriert werden kann.

Das erfindungsgemäße Verfahren kann zur großtechnischen Herstellung (> 100 kt/a) von Isopropanol aus Aceton eingesetzt werden. Die Bildung von Nebenprodukten wird nahezu vollständig vermieden, eine aufwendige Aufarbeitung entfällt somit.

Gegenstand der vorliegenden Erfindung ist daher ein Verfahren zur Hydrierung von Aceton zu Isopropanol, wobei eine Flüssigphasenhydrierung mit mindestens zwei Verfahrensstufen durchgeführt wird und ein nickelhaltiger Katalysator auf einem neutralen Träger eingesetzt wird.

Bei der Hydrierung von Aceton können die folgenden Reaktionen ablaufen:

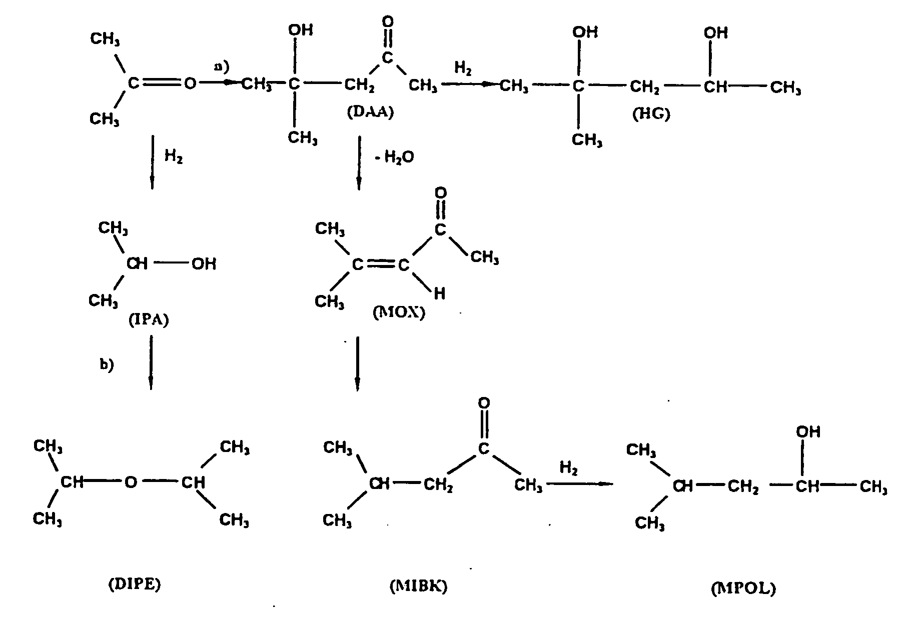

Nach der alkalisch katalysierten Aldokondensation a) von Aceton zum Diacetonalkohol (DAA) führt eine Wasserabspaltung zum 4-Methyl-3-penten-2-on (Mesityloxid, MOX). Die Hydrierung der Zwischenkomponente MOX führt über das 4-Methyl-2-pentanon (Methylisobutylketon, MIBK) zum 4-Methyl-2-pentanol (MPOL). DAA kann aber auch direkt zum Hexylenglykol (HG) hydriert werden. Das Zielprodukt IPA kann weiterhin unter Wasserabspaltung b) zum unerwünschten Diisopropylether (DIPE) weiterreagieren.

Ein geeigneter Katalysator sollte möglichst neutral reagieren, um nicht die unerwünschte Nebenreaktion des IPA, die Aldolkondensation und nachfolgende Wasserabspaltung, zu katalysieren.

Einige der oben genannten Nebenreaktionen laufen unter Wasserabspaltung ab. Zur Unterbindung dieser Nebenreaktionen, d. h. zur Selektivitätserhöhung ist daher ein geringer Wasserzusatz denkbar. Dieser, für spezielle Anwendungen des Isopropanols unerwünschte Wasserzusatz verbleibt im Produktgemisch und muß ggf. entfernt werden.

Die vorliegende Erfindung ermöglicht dagegen die Hydrierung von Aceton mit einem sehr geringen Wasseranteil. Dies ist umso überraschender, da in der vorgenannten Literatur ein Zusatz von Wasser in den Eduktstrom zur Selektivitätserhöhung bzw. Reduzierung der Nebenproduktbildung nötig ist.

Mit dem erfindungsgemäßen Verfahren kann Aceton mit einem Wassergehalt von kleiner oder gleich 1.0, bevorzugt kleiner oder gleich 0.5, ganz besonders bevorzugt 0.2 Gew.-% zu Isopropanol hydriert werden.

Die in großtechnischen Prozessen angestrebten hohen Umsätze können im vorliegenden Fall entweder durch Kreislaufreaktoren oder hintereinandergeschaltete, kaskadierende Reaktoren erreicht werden.

Durch das erfindungsgemäße mehrstufige Verfahren kann Aceton zu Isopropanol hoher Reinheit hydriert werden. Die einzelnen, parallel und/oder kaskadiert angeordneten Verfahrensstufen können als Kreislauf- oder Rohrreaktoren ausgeführt sein.

Die Reaktionsbedingungen können in weiten Grenzen variiert werden; die Flüssigphasenhydrierung kann bei einer Temperatur von 60 bis 140, bevorzugt 70 bis 130 °C und einem Druck von 20 bis 50, bevorzugt 25 bis 35 bar durchgeführt werden. Die Temperatur- und Druckverhältnisse können sich in den verschiedenen Verfahrensstufen unterscheiden.

In der Regel wird mit einem Wasserstoffüberschuß gearbeitet; das molare Verhältnis von Wasserstoff zu Aceton kann 1.5 zu 1 bis 1 zu 1 betragen.

In einer speziellen Ausführungsform des erfindungsgemäßen Verfahrens werden zwei Verfahrensstufen eingesetzt, wobei der Reaktor der 1. Verfahrensstufe als Kreislaufreaktor und der Reaktor der 2. Verfahrensstufe als Rohrreaktor ausgeführt werden kann.

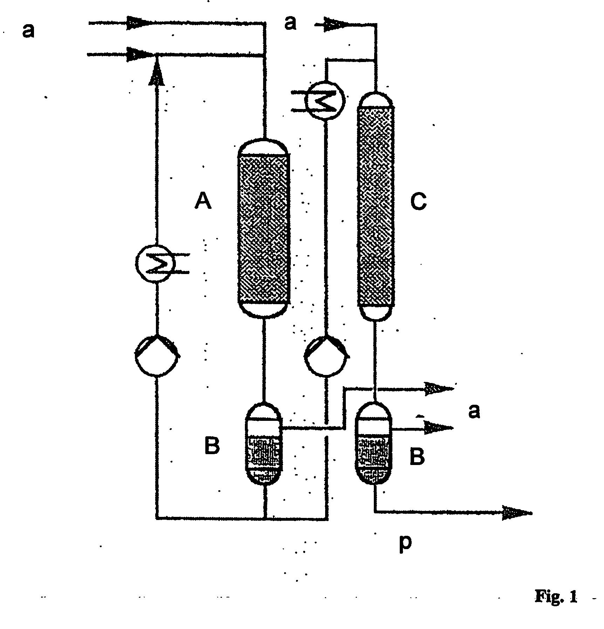

Ein vereinfachtes Ablaufschema des erfindungsgemäßen Verfahren mit einigen optionalen Bauteilen ist in Fig. 1 skizziert.

Durch den vorgeschalteten Kreislaufreaktor A mit Produktrückführung wird ein Großteil des geforderten Umsatzes erreicht. Dieser Reaktor arbeitet auf einem hohen Konzentrationsniveau und kann mit einem kleinen Kreislaufverhältnis betrieben werden. Der Reaktionsaustrag des Kreislaufreaktors kann zwischengekühlt werden (B). Die Hydrierung zum Endumsatz erfolgt in einem als Rohrreaktor betriebenen Schachtofen (C) ohne Produktrückführung. Mit a) sind die Wasserstoffzu- bzw. -abfuhrleitungen, mit p) die Produktleitung gekennzeichnet. Beide Reaktoren (A und C in Fig. 1) sind als adiabatische Reaktoren konzipiert.

Die Starttemperatur der 1. Verfahrensstufe beträgt zwecksmäßig 50 bis 90 °C, der Gesamtdruck 10 bis 30 bar. Sollte eine höhere Anfangsaktivität des Katalysators vorliegen, so kann entweder die Starttemperatur gesenkt oder das Kreislaufverhältnis im ersten Reaktor erhöht und damit die gewünschte Austrittstemperatur, die der Eingangstemperatur des zweiten Reaktor entsprechen kann, eingestellt werden.

Der Reaktor der 1. Verfahrensstufe kann als Kreislaufreaktor mit einem Kreislaufverhältnis von 6 bis 10 betrieben werden. Der Acetonanteil im Kreislaufstrom sinkt um 8 bis 20 Gew.-%, während der Isopropanolanteil um den entsprechenden Anteil steigt. Der Hydriervorgang ist exotherm, so daß im oder nach dem Kreislaufreaktor eine Kühlung vorgesehen sein sollte. Die Flüssigphasenhydrierung der 1. Verfahrensstufe kann bei einer Temperatur von 60 bis 130 °C, bevorzugt 80 bis 120 °C und einem Druck von 20 bis 50, bevorzugt 25 bis 35 bar durchgeführt werden.

Die mit einer Rohrreaktorcharakteristik betriebene 2. Verfahrensstufe kann bei einer Tempratur von 60 bis 140 °C, bevorzugt 70 bis 130 °C und einem Druck von 20 bis 50 bar durchgeführt werden.

In den Verfahrensstufen können die gleichen Hydrierkatalysatoren eingesetzt werden. Es bieten sich handelsübliche Hydrierkatalysatoren Ni als aktive Komponente auf einem neutralen Trägermaterialien an. Im erfindungsgemäßen Verfahren haben sich nickelhaltige Katalysatoren, z. B. mit ca. 10 Gew.-% Nickel auf einem neutralen Träger bewährt.

Das Trägermaterial des Katalysators sollte in jedem Fall neutral sein. Neutrale Trägermaterialien sind z. B. α-Al₂O₃, TiO₂, ZrO₂ oder Mullit.

Das erfindungsgemäße Verfahren liefert Isopropanol von hoher Reinheit. Die Gesamtkonzentration der durch die Hydrierung gebildeten Nebenprodukte wie 4-Methyl-3-penten-2-on, 4-Methyl-2-pentanol, Diacetonalkohol, Hexylenglykol und Diisopropylether kann unter 300, bevorzugt unter 200, ganz besonders bevorzugt unter 100 ppm liegen.

Das mehrstufige Reaktorkonzept bietet weitere Vorteile durch eine hohe Flexibilität. Kreislaufverhältnis, Druck und Temperatur können in den Reaktoren unabhängig voneinander eingestellt werden. Sollte die Katalysatoraktivität in einem Reaktor nachlassen, so kann z. B. eine entsprechend höhere Temperatur im Folgereaktor zugelassen werden.

Bei der Dimensionierung der Reaktoren sollte für eine gute Flüssigkeitsverteilung bzw. eine hohe Gasaustauschfläche gesorgt sein. Dies kann durch einen geeigneten Flüssigkeitsverteiler wie z. B. Raschigringe, Drahtgeflecht oder Sulzer-Mischer sowie eine hinreichend hohe Querschnittsbelastung von mindestens 30 m³/(m²·h) sichergestellt werden.

Das folgende Beispiel soll die Erfindung näher beschreiben, ohne jedoch ihren Anwendungsbereich einzuschränken.

### Beispiel:

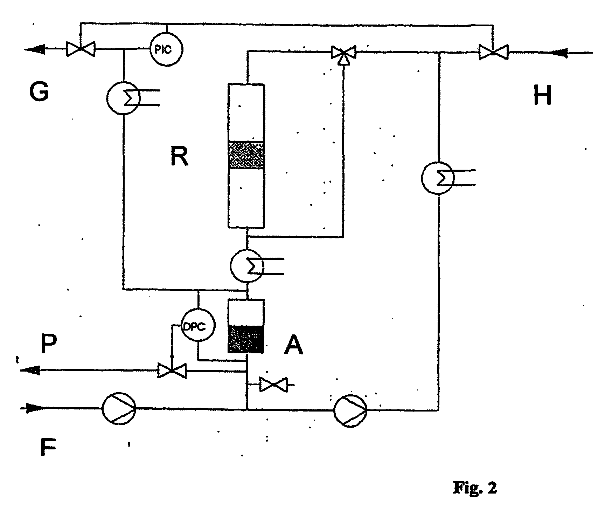

Es wurde eine Versuchsanlage gemäß Fig. 2 eingesetzt.

Bei den diskontinuierlich durchgeführten Versuchen wird das Edukt F in dem Trennbehälter A vorgelegt und im Umgang im Kreislauf gepumpt. Anschließend wird die Apparatur auf die gewünschten Reaktionsbedingungen gebracht. Zu Beginn der Reaktion wird der Kreislauf auf Reaktor R geschaltet. Nach ca. 5 Minuten haben sich konstante Temperatur- und Druckwerte eingestellt, und es werden die ersten Produktproben gezogen. Die Wasserstoffzu- bzw. -abfuhr erfolgt über die Leitungen G bzw. H. Durch das Umpumpen des Produktes werden bei geeigneter Katalysatoreinwaage differentielle Umsätze bei einmaligem Durchgang durch die Katalysatorschüttung realisiert. Des weiteren wird ein isothermer Betrieb garantiert, welcher die kinetische Auswertung der Versuche vereinfacht. Durch Probennahme zu verschiedenen Versuchzeiten kann ein Konzentrationskontaktzeitverlauf aufgenommen werden. Solche Versuche entsprechen dem reaktionstechnischen Modell eines diskontinuierlich betriebenen Rührkessels bzw. eines Rohrreaktors.

Es kam ein nickelhaltiger Katalysator (10 Gew.-% Nickel) auf einem neutralen α-Al₂O₃-Träger zum Einsatz.

### Versuchsergebnisse:

| **Kreislaufreaktor der 1. Verfahrensstufe** | | | |
|---|---|---|---|
| Eingangstemperatur | | 70 °C | |
| Ausgangstemperatur | | 115 °C | |
| Kreislaufverhältnis | | 1 : 8 | |
| Querschnittbelastung | | 220 m/h | |

| | | **Eingang Gew.-%** | **Ausgang Gew.-%** |
|---|---|---|---|
| | Aceton | 22.2 | 12.5 |
| | Isoproanol | 77.8 | 87.5 |

| **Rohrreaktor der 2. Verfahrensstufe** | | | |
|---|---|---|---|
| Eingangstemperatur | | 70 °C | |
| Ausgangstemperatur | | 126 °C | |
| Querschnittsbelastung | | 38 m/h | |

| | | **Eingang Gew.-%** | **Ausgang Gew.-%** |
|---|---|---|---|
| | Aceton | 12.5 | 0.54 |
| | Isopropanol | 87.5 | 99.45 |
| | Nebenprodukte | | < 100 ppm |

Nebenprodukte sind: Methylisobutyketon, 4-Methyl-2-pentanol, Hexylenglykol und weitere, nicht bestimmte Hochsieder.

## Patentansprüche

1. Verfahren zur Hydrierung von Aceton zu Isopropanol durch Flüssigphasenhydrierung von Aceton mit mindestens zwei Verfahrensstufen,
**dadurch gekennzeichnet,**
**dass** zur Flüssigphasenhydrierung ein nickelhaltiger Katalysator auf einem neutralen Träger eingesetzt wird.

2. Verfahren nach Anspruch 1,
**dadurch gekennzeichnet,**
**dass** die Flüssigphasenhydrierung bei einer Temperatur von 60 bis 140°C und einem Druck von 20 bis 50 bar durchgeführt wird.

3. Verfahren nach Anspruch 1 oder 2,
**dadurch gekennzeichnet,**
**dass** die Gesamtkonzentration der durch die Hydrierung gebildeten Nebenprodukte 300 ppm nicht überschreitet.

4. Verfahren nach einem der Ansprüche 1 bis 3,
**dadurch gekennzeichnet,**
**dass** Aceton mit einem Wassergehalt unter 1,0 Gew.-% hydriert wird.

5. Verfahren nach Anspruch 1,
**dadurch gekennzeichnet, dass** ein nickelhaltiger Katalysator auf einem α-Al₂O₃-Träger eingesetzt wird.

## Claims

1. A process for the hydrogenation of acetone by liquid phase hydrogenation of acetone in at least two hydrogenation process stages, **characterized in that** a nickel-containing catalyst on a neutral carrier is employed for the liquid phase hydrogenation.

2. The process according to claim 1, **characterized in that** the liquid phase hydrogenation is conducted at a temperature of from 60 to 140°C and a pressure of from 20 to 50 bar.

3. The process according to claim 1 or 2, **characterized in that** the total concentration of byproducts formed during the hydrogenation does not exceed 300 ppm.

4. The process according to any of claims 1 to 3, **characterized in that** acetone having a water content of less than 1.0 weight percent is hydrogenated.

5. The process according to claim 1, **characterized in that** a nickel containing catalyst on an α-Al₂O₃ carrier is employed.

## Revendications

1. Procédé d'hydrogénation d'acétone en isopropanol par hydrogénation en phase liquide d'acétone, comprenant au moins deux étapes de procédé, **caractérisé en ce que**, pour l'hydrogénation en phase liquide, on met en oeuvre un catalyseur au nickel sur un support neutre.

2. Procédé suivant la revendication 1, **caractérisé en ce que** l'hydrogénation en phase liquide est entreprise à une température de 60 à 140C° et sous une pression de 20 à 50 bar.

3. Procédé suivant la revendication 1 ou 2, **caractérisé en ce que** la concentration totale des sous-produits formés par l'hydrogénation n'excède pas 300 ppm.

4. Procédé suivant l'une quelconque des revendications 1 à 3, **caractérisé en ce que** l'on hydrogène de l'acétone ayant une teneur en eau de moins de 1,0% en poids.

5. Procédé suivant la revendication 1, **caractérisé en ce que** l'on met en oeuvre un catalyseur au nickel sur un support d'α-Al₂O₃.
